# FASCICULE DE BREVET EUROPEEN

(11) **EP 0 891 767 B1**
(45) Date de publication et mention de la délivrance du brevet: **18.07.2001**
(21) Numéro de dépôt: 98401459.7
(22) Date de dépôt: 15.06.1998
(51) Int. Cl.: A61K 7/48

(54) **Composition brillante contenant des huiles aromatiques épaissies par un alkyléther de polysaccharide**
Glänzende Zusammensetzung enthaltend aromatische Öle und ein Alkylether-Polysaccharid als Verdicker derselben
Glossy composition containing aromatic oils thickened with an alkylether of a polysaccharide

(30) Priorité: 08.07.1997 FR 9708675
(43) Date de publication de la demande: 20.01.1999
(73) Titulaire: L'OREAL, 75008 Paris (FR)
(72) Inventeur: Pinzon, Carlos, Hackensack, New Jersey 07601 (US); Arnaud, Pascal, 94240 L'Hay les Roses (FR)
(74) Mandataire: Lhoste, Catherine

(56) Documents cités:
- EP-A- 0 708 114
- EP-A- 0 781 780
- EP-A- 0 804 922
- RESEARCH DISCLOSURE, GB, Avril 1996 XP000596112
- RESEARCH DISCLOSURE, GB, Octobre 95 XP000549119

## Description

La présente invention se rapporte à une composition brillante contenant des huiles aromatiques épaissies par un nouvel épaississant, destinée en particulier aux domaines cosmétique et/ou dermatologique. Plus spécialement l'invention se rapporte à une composition de maquillage de la peau et/ou des muqueuses telles que les lèvres et l'intérieur des paupières inférieures des yeux d'êtres humains. L'invention se rapporte encore à une utilisation de cette composition et à un procédé cosmétique pour le traitement, le maquillage et/ou le soin de la peau et/ou des muqueuses. Cette composition peut se présenter sous forme de stick ou coupelle, de pâte molle ou encore de liquide huileux plus ou moins épaissi. Elle constitue en particulier un produit à lèvres, un fond de teint, un eye liner, un produit anti-cernes, un fard à paupières ou encore un produit de protection solaire.

Dans les produits cosmétiques ou dermatologiques, il est courant de trouver une phase grasse liquide épaissie ; ceci est notamment le cas dans les onguents, les gels de soins ou gommants anhydres, les compositions solides comme les déodorants, les baumes et les rouges à lèvres. L'épaississement des huiles (ou des phases liquides à température ambiante) permet en particulier de limiter l'exsudation des huiles des compositions solides et, en plus, dans le cas des rouges à lèvres de limiter voire éliminer totalement la migration du film coloré dans les rides et ridules autour des lèvres. Ce problème de migration existe aussi, à un niveau moindre, dans les produits contours des yeux.

Pour remédier à ces problèmes, on a habituellement recourt à des cires ou des charges. Malheureusement, ces cires et charges ont tendance à matifier la composition, ce qui n'est pas toujours souhaitable. En effet, les femmes sont de plus en plus à la recherche d'un rouge à lèvres brillant qui n'exsude pas et ne migre pas.

Une des techniques connues pour augmenter la brillance d'un produit de maquillage ou de soin est d'augmenter la proportion de la phase huileuse au détriment de la phase particulaire, cette dernière devant être par ailleurs la mieux dispersée possible. En effet, une mauvaise dispersion des particules pigmentaires peut conduire à un film non homogène sur les lèvres, conférant un aspect peu esthétique. De plus une trop faible quantité de pigments conduit souvent à un film peu couvrant. En outre, la brillance de la composition a tendance à décroître dans le temps, notamment à cause de la mauvaise tenue du film dans le temps.

Il est par ailleurs connu d'épaissir les huiles (ou de façon générale les phases grasses liquides à température ambiante) avec des épaississants polymériques et notamment des polyoléfines. Malheureusement, ces épaississants d'huiles connus doivent être utilisés en grande quantité pour obtenir un épaississement efficace. Or, une trop grande quantité d'épaississant confère à la composition, lorsque cette dernière est destinée au domaine cosmétique, des propriétés cosmétiques insuffisantes, et notamment un toucher collant et un manque de glissant, ces inconvénients pouvant être très gênants, voire rédhibitoires.

De manière surprenante et inattendue, la demanderesse a découvert qu'il était possible d'obtenir des compositions cosmétiques brillantes et stables sous forme de gel, stick ou pâte souple présentant des propriétés cosmétiques satisfaisantes, grâce à une phase grasse liquide particulière épaissie par un épaississant particulier. Cette composition permet notamment de résoudre tous les inconvénients mentionnés ci-dessus.

La présente invention a donc pour objet une composition contenant une phase grasse liquide contenant au moins une huile comportant une structure chimique pourvue d'au moins un groupement aromatique, un épaississant de ladite phase grasse et une charge particulaire, cet épaississant comprenant au moins un alkyléther de polysaccharide formé de motifs comportant au moins deux cycles osidiques différents, chaque motif comportant au moins un groupe hydroxyle substitué par une chaîne alkyle hydrocarbonée saturée ou non.

Grâce à cet épaississant particulier et à cette sélection de phase grasse, la composition selon l'invention permet l'obtention d'un film brillant sur la peau ou les lèvres. Il est, en outre, possible d'obtenir, en association avec des ingrédients connus pour rigidifier ou solidifier la composition, un produit solide sous forme de pâte souple ou de stick présentant des propriétés cosmétiques améliorées par rapport aux compositions épaissies de l'art antérieur. En particulier, il est possible d'obtenir un bâton de rouge à lèvres, un stick anti-cernes ou un fond de teint coulé, doux à l'application ayant un aspect brillant et dans lequel la phase grasse liquide n'exsude pas. De plus, dans le cas plus particulier d'un rouge à lèvres, les huiles chargées en pigments ne migrent pas dans les rides et ridules des encoignures des lèvres.

Bien que l'invention soit particulièrement bien adaptée au domaine cosmétique, elle s'applique à tout domaine nécessitant d'obtenir des compositions épaisses brillante voire des compositions solides brillantes et notamment dans les domaines, vétérinaire, dermatologique, pharmaceutique, ou encore du bois (stick de pigments, d'huiles et de cires pour la restauration de meubles).

Dans l'épaississant de l'invention, on entend par « chaîne alkyle hydrocarbonée » une chaîne linéaire ou ramifiée, comportant de 1 à 24, de préférence de 1 à 10, mieux de 1 à 6 et plus spécialement de 1 à 3 atomes de carbone. En particulier, la chaîne alkyle est choisie parmi les chaînes saturées et notamment methyle, éthyle, éthényle, n-propyle, propényle, isopropyle, n-butyle, isobutyle, tertiobutyle, n-pentyle. Ces alkyléthers peuvent être fabriqués comme décrits dans les documents EP-A-281 360, EP-A-708 114, EP-A-281360.

Selon un mode de réalisation préféré de l'invention, l'alkyléther de polysaccharide a un poids moléculaire moyen en poids supérieur à 100 000, et de préférence supérieur à 200 000. Ce poids moléculaire moyen peut aller jusqu'à 1 million. Cet alkyléther peut comporter de un à six et mieux de deux à quatre groupes hydroxyle par motif, substitués par une chaîne alkyle hydrocarbonée saturée ou non.

Les cycles osidiques sont notamment choisis parmi le mannose, le galactose, le glucose, le furanose, le rhamnose, l'arabinose.

Selon un mode préféré de réalisation de l'invention, l'alkyléther de polysaccharide est un alkyléther d'une gomme et plus particulièrement d'une gomme globalement non ionique, c'est-à-dire comportant peu ou pas de groupe ionique. Comme gommes appropriées, on peut citer par exemple la gomme guar dont le motif comprend un galactose et un mannose, la gomme de caroube dont le motif comprend un galactose et un mannose, la gomme de karaya qui est un mélange complexe de rhamnose, galactose et acide galacturonique, la gomme adragante qui est un mélange complexe d'arabinose, galactose et acide galacturonique.

Selon un mode de réalisation préféré de l'invention, l'alkyléther de polysaccharide est un dérivé de gomme guar. Ainsi, avantageusement l'alkyéther est un galactomannane alkylé de chaîne alkyle en C₁ à C₆ et mieux en C₁ à C₃ et plus particulièrement la guar éthylée ayant un degré de substitution de 2 à 3 et notamment d'environ 2,5 à 2,8, telle que décrite dans les documents RD 95378007 (octobre 1995) et EP-A-708114. Cette gomme est en particulier celle vendue par la société Aqualon sous le nom N-HANCE-AG 200® et N-HANCE-AG 50®.

La concentration en alkyléther dépend de la forme galénique, de la consistance recherchées pour la composition ainsi que de la quantité d'huile à épaissir. En particulier le rapport en poids de la quantité de phase grasse liquide sur la quantité d'épaississant est choisi par exemple dans la gamme allant de 5 à 500. La composition selon l'invention peut contenir par exemple une quantité d'alkyléther de polysaccharide allant de 0,2 à 16 % du poids total de la composition, et de préférence de 0,5 à 16 % et mieux de 1,5 à 8 %.

La composition de l'invention peut contenir une ou plusieurs huiles à groupement aromatique. Ces huiles peuvent avoir un squelette hydrocarboné ou siliconé avec éventuellement des groupements fluorés. La structure chimique de ces huiles peut comporter un ou plusieurs radicaux aryle ou aralkyle qui peuvent être mono-ou multi-substitués. Ces radicaux aryle ou aralkyle peuvent avoir de 6 à 60 atomes de carbone.

Selon l'invention, les huiles à squelette hydrocarboné et à groupement aromatique contiennent au moins 8 atomes de carbone et ne sont pas des solvants du type benzène ou toluène.

A titre d'exemples d'huiles à squelette hydrocarboné possédant un radical aryle monosubstitué, on peut citer les esters de l'acide benzoïque tels que l'éthyl hexyle benzoate, le C₁₂-C₁₅ alkylbenzoate, l'octyldodécyl benzoate ou le dibenzoate de dipropylène glycol et leurs mélanges.

Comme exemple d'huiles à squelette hydrocarboné possédant un radical aryle multisubstitué, on peut citer les esters de l'acide trimellitique (ou les trialkyl trimellitate) avec des chaînes alkyle en C₁ à C₂₂ tel que le tridécyl trimellitate ; les esters de l'acide para-méthoxycinnamique comme le para-méthoxycinnamate d'éthyl-hexyle et le para-méthoxycinnamate de 2-étoxy éthyle ; les esters de l'acide diméthyl para-aminobenzoïque tels que l'octyl diméthyl PABA (ou 2 éthylhexyldiméthyl para-amino benzoate) ; les esters de l'acide salicylique comme le 2-éthylhexyl salicylate ; les esters de l'acide anthranilique tels que le menthyl anthranilate ; et leurs mélanges.

A titre d'exemples d'huiles à squelette siliconé possédant un ou plusieurs radicaux aryle monosubstitués, on peut citer les silicones phénylées présentant notamment la formule suivante : 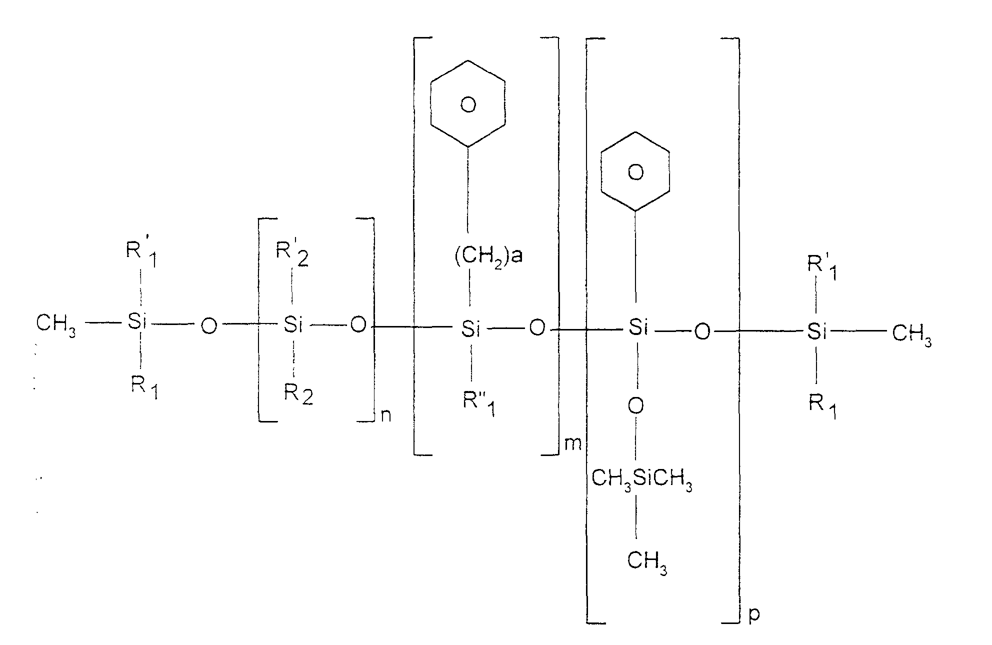 dans laquelle
. R₁, R'₁ et R"₁ représentent indépendamment un radical alkyle linéaire ou ramifié, saturé ou insaturé en C₁ à C₃₀, un radical aryle, un radical phényle ou un radical aralkyle en C₇ à C₆₀,
. R₂ et R'₂ représentent indépendamment un radical alkyle linéaire ou ramifié. saturé ou insaturé en C₁ à C₃₀
. n est un nombre entier allant de 0 à 150,
. m est un nombre entier allant de 0 à 200,
. p est un nombre entier allant de 0 à 200, et
. a est un nombre entier allant de 0 à 10,
sous réserve que le rapport n/(n + m + p) soit au plus égal à 0,85 et que la somme n + m + p va de 1 à 200 et que si m = p = 0 alors R'₁ est un radical aryle ou aralkyle.

De préférence, R₁ et R'₁ sont égaux et représentent un radical méthyle, éthyle, propyle, isopropyle, décyle, dodécyle ou octadécyle, ou encore un radical phényle, tolyle, benzyle ou phénéthyle.

Avantageusement, R₂ et R'₂ sont égaux et représentent un radical méthyle, éthyle, propyle, isopropyle, décyle, dodécyle ou octadécyle, et mieux méthyle.

De préférence, R"₁ est un radical méthyle, éthyle, propyle, isopropyle, décyle, dodécyle ou octadécyle, ou encore un radical phényle, tolyle, benzyle ou phénéthyle. En particulier, il est égal à méthyle ou phényle.

Parmi ces silicones phénylées, on peut citer notamment les phényl triméthicones, les phényltriméthylsiloxydiphényl siloxanes, les diphénylméthyldiphényltrisiloxanes, les 2-phényléthyltriméthylsiloxysilicates, les diphénylméthyldiméthyltrisiloxanes, les diphényl diméthicones, les phényl diméthicones, les polyméthylphényl siloxanes, les phényltriméthicones et leurs mélanges. A titre d'exemple de silicones phénylées commerciales, on peut citer l'huile Belsil PDM1000 vendue par la société Wacker, les huiles DC556, SF558 ou DC704 vendues par la société Dow Corning, l'huile Abil AV8853 de Goldschmidt, l'huile Silbione 70641V200 ou l'huile Silbione 70633V30 vendues par la société Rhône Poulenc, ou encore celles vendues par la société PCR sous les noms 15M30, 15M40,15M50,15M60.

Comme autres huiles de silicones à groupement aromatique utilisables dans l'invention, on peut citer les résines modifiées par des groupements styryle telles que celles décrites dans les brevets US-A-5338538 et US-A-5397566 de Général Electric, et notamment les phényl éthyl triméthylsiloxysilicates comme celles vendues sous la dénomination 1170-3100. On peut aussi utiliser des huiles de silicone fluorée à groupement aromatique et notamment perfluorée comme celles décrites dans les documents EP-A-811369 et EP-A-829254; les radicaux perfluorés sont des radicaux pendants ayant de 1 à 12 atomes de carbone.

La composition selon l'invention peut comprendre de 1 à 99,8 % en poids, de préférence de 5 à 80%, d'huiles à groupement aromatique.

La phase particulaire de la composition de l'invention est généralement présente à raison de 0,05 à 35 % du poids total de la composition, de préférence de 0,5 à 20 %, et qui peut comprendre des pigments et/ou des nacres et/ou des charges habituellement utilisés dans les compositions cosmétiques ou dermatologiques. Cette charge peut conduire à une composition colorée, blanche ou incolore.

Par pigments, il faut comprendre des particules blanches ou colorées, minérales ou organiques, insolubles dans la phase grasse liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments peuvent être présents dans la composition à raison de 0,05 à 25 % du poids de la composition finale, et de préférence à raison de 2 à 15 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (DC Red N°7), aluminium.

Les nacres peuvent être présentes dans la composition à raison de 0 à 20 % du poids total de la composition, de préférence à un taux élevé de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 35 % du poids total de la composition, de préférence 0,5 à 15 %. On peut notamment citer le talc, le mica, le kaolin, les poudres de Nylon (Orgasol notamment) et de polyéthylène, le Téflon, l'amidon, le nitrure de bore, des microsphères de copolymères telles que l'Expancel (Nobel Industrie), le polytrap (Dow Corning) et les microbilles de résine de silicone (Tospearl de Toshiba, par exemple).

Avantageusement, la phase grasse liquide contient, une ou plusieurs autres types d'huile que les huiles à groupement aromatique. Ces huiles peuvent être des huiles hydrocarbonées et/ou siliconées et/ou fluorées. Ces huiles peuvent être d'origine animale, végétale, minérale ou synthétique.

Comme huiles autres que celles à groupement aromatique, utilisables dans l'invention, on peut citer notamment :
- les huiles hydrocarbonées d'origine animale telles que le perhydrosqualène ;
- les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;
- les hydrocarbures linéaires ou ramifiés, d'origine minérale ou synthétique tels que les huiles de paraffine et leurs dérivés, la vaseline, les polydécènes, l'huile de Purcellin, le polyisobutène hydrogéné tel que le parléam ;
- les esters et les éthers de synthèse notamment d'acides gras comme les huiles de formule R₃COOR₄ dans laquelle R₃ représente le reste d'un acide gras supérieur comportant de 7 à 29 atomes de carbone et R₄ représente une chaîne hydrocarbonée contenant de 3 à 30 atomes de carbone comme par exemple l'huile de Purcellin, le myristate d'isopropyle, le palmitate d'éthyl-2hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ;
- des alcools gras ayant de 12 à 26 atomes de carbone comme l'octyldodécanol, le 2-butyloctanol, le 2-hexyldécanol, le 2-undécylpentadécanol, l'alcool oléique ;
- les huiles fluorées partiellement hydrocarbonées et/ou siliconées comme celle décrites dans le document JP-A-2-295912 ;
- les huiles siliconées comme les polyméthylsiloxanes (PDMS) volatiles ou non, linéaires ou cycliques, liquides ou pâteux à température ambiante ;
- leurs mélanges.

Ces huiles peuvent représenter de 0 à 99% en poids par rapport à la phase grasse liquide.

Comme huile de silicone volatile utilisable dans l'invention, on peut citer les silicones linéaires ou cycliques ayant de 2 à 7 atomes de silicium, ces silicones comportant éventuellement des groupes alkyle ayant de 1 à 10 atomes de carbone. Ainsi, ces silicones sont notamment l'hexaméthyldisiloxane, la cyclopenta- ou cyclotétra- ou cyclohexa-diméthylsiloxane. Ces huiles volatiles peuvent représenter de 0 à 50% du poids total de la composition.

La composition selon l'invention peut, de plus, comprendre tous les ingrédients classiquement utilisés dans les domaines concernés et plus spécialement dans les domaines cosmétique et dermatologique. Ces ingrédients sont en particulier choisis parmi les conservateurs, les épaississants de phase aqueuse ou grasse différents de celui de l'invention, les parfums, les tensioactifs, les antioxydants, les cires et leurs mélanges. La composition selon l'invention peut contenir également des vésicules lipidiques de type ionique et/ou non ionique. Les quantités de ces différents ingrédients sont celles classiquement utilisées dans les domaines concernés et par exemple de 0,01 % à 20 % du poids total de la composition. La nature de ces ingrédients et leur proportion doit être compatibles avec l'obtention de compositions selon l'invention, stables épaissies et brillantes. La composition peut aussi contenir de l'eau à une concentration allant de 0 à 95% du poids total de la composition.

La composition de l'invention peut comprendre, avantageusement une phase grasse solide ou pâteuse, contenant une ou plusieurs gommes et/ ou une ou plusieurs cires. Ces gommes ou cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.

Comme cire utilisable dans la composition de l'invention, on peut citer, la lanoline, la cire d'abeilles, la cire de Carnauba ou de Candellila, la paraffine, les cires de lignite ou microcristalline, la cérésine ou l'ozokérite ; les cires synthétiques comme les cires de polyéthylène, les cires de silicones comme les alkyl ou alkoxy-diméticone ayant de 16 à 45 atomes de carbone.

La nature et la quantité de ces gommes ou cires sont fonction des propriétés mécaniques et de textures recherchées. A titre indicatif, la composition peut contenir de 0 à 50 % en poids de cires, par rapport au poids total de la composition et mieux de 5 à 30 %.

La composition de l'invention peut se présenter sous toutes les formes galéniques normalement utilisées pour une application topique, et notamment sous forme d'un gel huileux, d'une émulsion huile-dans-eau ou eau-dans-huile, d'une dispersion d'huile dans de l'eau grâce à des vésicules, les vésicules étant situées à l'interface huile/eau. Cette composition peut avoir l'aspect d'une crème, d'une pommade, d'une pâte souple, d'un onguent, d'un solide coulé ou moulé et notamment de stick ou de coupelle.

La composition selon l'invention peut être avantageusement utilisée pour le traitement, le maquillage ou le soin de la peau et/ou des muqueuses selon la nature des actifs utilisés. En particulier, la composition de l'invention peut être un bâton de rouge à lèvres, un baume à lèvres, un brillant à lèvres (gloss en terminologie anglo-saxonne) utilisable tel quel ou pour appliquer sur un film de rouge à lèvres notamment pour en augmenter sa brillance et/ou en diminuer la migration des huiles (top coat en terminologie anglo-saxonne). Elle peut aussi constituer un fond de teint solide, un produit anti-cernes ou contours des yeux, un eye liner, un mascara, une ombre à paupières ou encore un produit de protection solaire ou un produit de soin ou de nettoyage de la peau tel que les produits gommant. Ces compositions peuvent, en outre, contenir des actifs cosmétiques ou dermatologiques, en vue, notamment d'apporter un aspect soin ou traitant à la composition. Ainsi la composition peut contenir des vitamines et autres actifs lipophiles (lanoline, filtre UVA) ou hydrophiles (hydratants comme la glycérine).

Aussi, l'invention a encore pour objet une utilisation cosmétique de la composition ci-dessus pour soigner et/ou maquiller la peau et/ou les muqueuses et plus spécialement les lèvres ainsi qu'une utilisation de cette composition pour la préparation d'un onguent destiné à traiter la peau et/ou les muqueuses et notamment les lèvres. L'invention a aussi pour objet un procédé de traitement cosmétique et/ou dermatologique de la peau et/ou des muqueuses et notamment des lèvres, consistant à appliquer sur la peau et/ou les muqueuses et notamment les lèvres la composition définie ci-dessus.

De façon plus spécifique, l'invention a pour objet un produit à lèvres ou fond de teint contenant une phase grasse liquide contenant au moins une huile comportant une structure chimique pourvue d'au moins un groupement aromatique, un épaississant de ladite phase grasse et une charge particulaire, cet épaississant comprenant au moins un alkyléther de polysaccharide tel que défini ci-dessus.

La composition de l'invention peut être obtenue par chauffage des différents constituants à la température de fusion des cires les plus élevées, puis coulage du mélange fondu dans un moule (coupelle ou doigt de gant). Elle peut aussi être obtenue par extrusion comme décrit dans la demande EP-A-667 146.

L'invention a encore pour objet l'utilisation, dans une composition contenant une phase grasse liquide et une charge particulaire, de l'association d'au moins une huile comportant une structure chimique pourvue d'au moins un groupement aromatique et d'un épaississant de ladite phase grasse, cet épaississant comprenant au moins un alkyléther de polysaccharide tel que décrit ci-dessus, afin de diminuer la migration de cette phase grasse liquide et/ou obtenir un film brillant.

L'invention a encore pour objet un procédé pour limiter la migration d'une composition de maquillage ou de soin de la peau ou des lèvres sur un substrat différent de la dite peau et desdites lèvres, contenant une phase grasse liquide et une charge particulaire, consistant à introduire dans la phase grasse liquide au moins une huile comportant une structure chimique pourvue d'au moins un groupement aromatique, cette phase grasse étant épaissie par un épaississant comprenant au moins un alkyléther de polysaccharide tel que défini précédemment.

Les exemples de compositions ci-après sont donnés à titre illustratif et sans caractère limitatif. Les quantités y sont données en % en poids.

### Exemple 1 de brillant à lèvres

| | |
|---|---|
| Guar éthylée ayant un degré de substitution d'environ 2,5 | 3,8 % |
| DC Red N°7 Calcium (laque) | 5,0 % |
| Phényl triméthicone (DC 556) | qsp 100 % |

Ce brillant à lèvres est obtenu en dispersant les pigments dans la phase grasse liquide à l'aide d'un broyeur tricylindre. La guar éthylée est ensuite ajoutée et solubilisée à 90°C pendant 3 heures dans le mélange. Ce dernier est ensuite refroidi jusqu'à la température ambiante. Le gel obtenu peut être appliqué au pinceau sur les lèvres. Il confère une coloration brillante rouge des lèvres, coloration et brillance durables dans le temps.

Ce brillant à lèvres à été testé en comparaison avec un brillant de l'art antérieur. Il a été jugé plus cosmétique et de tenue en brillance supérieure à celui de l'art antérieur.

### Exemples 2 à 6 de brillant à lèvres

Ces exemples se distinguent de celui de l'exemple 1 par la nature de l'huile phénylée. Ces exemples contiennent respectivement du phényltriméthylsiloxydiphénylsiloxane (SF558) ; du diphényl diméthicone (Silbione 70641V200) ; du diphénylméthyl diméthyltrisiloxane (DC704) ; du 2-phényl éthyl triméthylsiloxysilicate (1170-3100) ; du tridécyl trimellitate (DUB TMTD).

### Exemple 7 de brillant à lèvres

| | |
|---|---|
| Guar éthylée ayant un degré de substitution d'environ 2,5 | 1,9 % |
| DC Red N° 7 Calcium (laque) | 5,0 % |
| Phényltriméthicone (Belsil PDM1000) | qsp 100 % |

Cette composition est utilisable telle quel comme brillant à lèvres ou comme top coat.

### Exemple 8 : Stick de rouge à lèvres

| | |
|---|---|
| Guar éthylée ayant un degré de substitution de 2,5 | 4,00 % |
| Polyéthylène (cire) | 3,50 % |
| Tridécyl trimellitate | 81,00 % |
| Cire de Carnauba | 3,50 % |
| DC Red 7 calcium (laque) | 8,00 % |

On disperse les pigments dans l'huile puis on dissout la guar éthylée à 90°C pendant 3 heures. On ajoute les cires et on chauffe l'ensemble jusqu'à fusion totale des cires. Enfin, on coule le mélange obtenu dans un moule de forme appropriée pour obtenir un bâton.

### Exemples 9 et 10 : Sticks de rouge à lèvres

Ces exemples se distinguent de celui de l'exemple 9 par l'emploi d'une guar méthylée et d'une guar propylée de degré de substitution d'environ 2,7.

## Revendications

1. Composition contenant une phase grasse liquide, une phase particulaire et un épaississant de la phase grasse, caractérisée en ce que l'épaississant comprend au moins un alkyléther de polysaccharide formé de motifs comportant au moins deux cycles osidiques différents, chaque motif comportant au moins un groupe hydroxyle substitué par une chaîne alkyle hydrocarbonée saturée ou non et en ce que la phase grasse contient au moins une huile comportant une structure chimique pourvue d'au moins un groupement aromatique.

2. Composition selon la revendication 1, caractérisée en ce que deux à quatre groupes hydroxyle par motif sont substitués par une chaîne alkyle hydrocarbonée saturée.

3. Composition selon la revendication 1 ou 2, caractérisée en ce que la chaîne alkyle hydrocarbonée comporte de 1 à 24 atomes de carbone.

4. Composition selon l'une des revendications précédentes, caractérisée en ce que la chaîne alkyle hydrocarbonée comporte de 1 à 10 atomes de carbone.

5. Composition selon l'une des revendications précédentes, caractérisée en ce que la chaîne alkyle est choisie parmi les chaînes méthyle, éthyle, n-propyle, isopropyle, n-butyle, isobutyle, tertiobutyle.

6. Composition selon l'une des revendications précédentes, caractérisée en ce que les cycles osidiques sont choisis parmi le mannose, le galactose, le glucose, le furanose, le rhamnose, l'arabinose.

7. Composition selon l'une des revendications précédentes, caractérisée en ce que l'alkyléther de polysaccharide est un alkyléther d'une gomme choisie parmi la gomme guar, la gomme de caroube, la gomme de karaya, la gomme adragante et leurs mélanges.

8. Composition selon l'une des revendications précédentes, caractérisée en ce que l'alkyéther est un galactomannane alkylé de chaîne alkyle en C₁ à C₆ et mieux en C₁ à C₃.

9. Composition selon l'une des revendications précédentes, caractérisée en ce que l'alkyléther de polysaccharide est de la gomme guar à chaîne éthyle avec un degré de substitution de 2 à 3.

10. Composition selon l'une des revendications précédentes, caractérisée en ce que l'alkyléther de polysaccharide a un poids moléculaire moyen en poids supérieur à 200 000.

11. Composition selon l'une des revendications précédentes, caractérisée en ce que l'alkyléther de polysaccharide est présent en une quantité telle que le rapport (en poids) de la quantité de phase grasse liquide sur la quantité d'épaississant est choisi dans la gamme allant de 5 à 500.

12. Composition selon l'une des revendications précédentes, caractérisée en ce que l'alkyléther de polysaccharide est présent en une quantité allant de 0,2 à 16 % du poids total de la composition et mieux de 1,5 à 8 % du poids total de la composition.

13. Composition selon l'une des revendications précédentes, caractérisée en ce que l'huile à groupement aromatique comporte un squelette hydrocarboné ou siliconé avec éventuellement des groupements fluorés et au moins un radical aryle ou aralkyle ayant de 6 à 60 atomes de carbone

14. Composition selon l'une des revendications précédentes, caractérisée en ce que l'huile à groupement aromatique est une huile hydrocarbonée dont la structure contient au moins 8 atomes de carbone.

15. Composition selon l'une des revendications 1 à 13, caractérisée en ce que l'huile à groupement aromatique est une huile siliconée de formule : 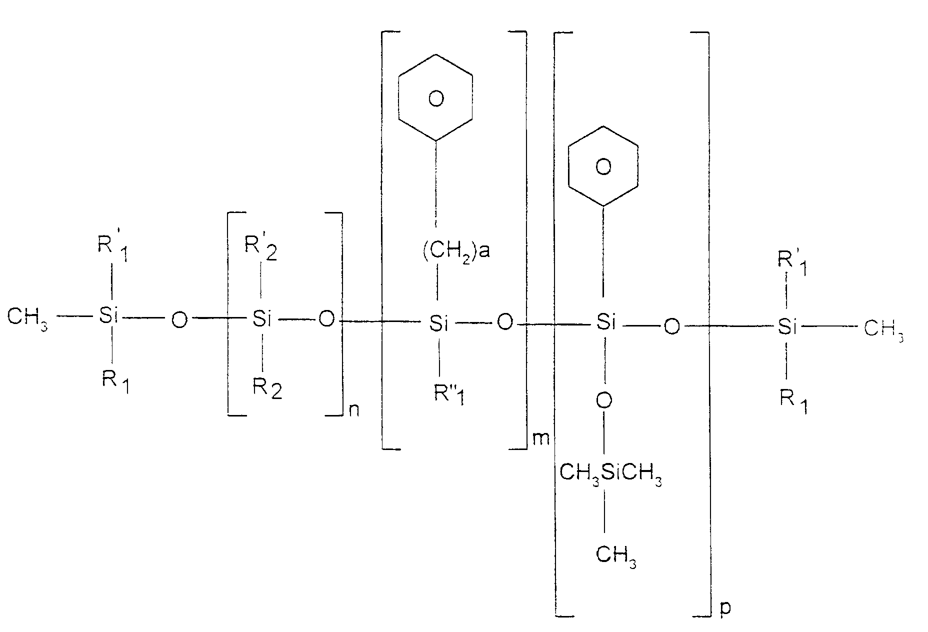 dans laquelle
. R₁, R'₁ et R"₁ représentent indépendamment un radical alkyle linéaire ou ramifié, saturé ou insaturé en C₁ à C₃₀, un radical aryle, un radical phényle ou un radical aralkyle en C₇ à C₆₀,
. R₂ et R'₂ représentent indépendamment un radical alkyle linéaire ou ramifié, saturé ou insaturé en C₁ à C₃₀
. n est un nombre entier allant de 0 à 150,
. m est un nombre entier allant de 0 à 200,
. p est un nombre entier allant de 0 à 200, et
. a est un nombre entier allant de 0 à 10,
sous réserve que le rapport n/(n + m + p) soit au plus égal à 0,85 et que la somme n + m + p va de 1 à 200 et que si m = p = 0 alors R'₁ est un radical aryle ou aralkyle.

16. Composition selon la revendication précédente, caractérisée en ce que R₁, R'₁ et R"₁ représentent un radical méthyle, éthyle, propyle, isopropyle, décyle, dodécyle, octadécyle, phényle, tolyle, benzyle, phénéthyle.

17. Composition selon la revendication 15 ou 16, caractérisée en ce que R₂ et R'₂ représentent un radical méthyle, éthyle, propyle, isopropyle, décyle, dodécyle, octadécyle.

18. Composition selon l'une des revendications précédentes, caractérisée en ce l'huile à groupement aromatique est choisie parmi les esters de l'acide trimellitique à chaînes alkyle en C₁ à C₂₂, les esters de l'acide benzoïque, les esters de l'acide para méthoxycinnamique, les esters de l'acide diméthyl para-amino benzoïque, les esters de l'acide salicylique, les esters de l'acide anthranilique, les phényl triméthicones, phényltriméthylsiloxydiphénylsiloxanes, les diphénylméthyl diméthyltrisiloxanes, les diphényl diméthicones, les phényl éthyl triméthylsiloxysilicates, les phényltriméthicones et les polyméthylphényl siloxanes et leurs mélanges.

19. Composition selon l'une des revendications précédentes, caractérisée en ce que l'huile à groupement aromatique est choisie parmi le tridécyl trimellitate, le para-méthoxycinnamate, les phényl triméthicones, les phényltriméthylsiloxydiphénylsiloxanes, les diphényl diméthicone, les diphénylméthyldiphényl trisiloxanes, les 2-phényléthyl triméthylsiloxysilicate et leurs mélanges.

20. Composition selon l'une des revendications précédentes, caractérisée en ce que la phase grasse contient au moins une huile autre que l'huile à groupement aromatique choisie parmi les huiles d'origine animale, végétale, minérale ou synthétique.

21. Composition selon l'une des revendications précédentes, caractérisée en ce que la phase particulaire est présente à raison de 0,05 à 35 % du poids total de la composition, de préférence de 0,5 à 20 %.

22. Composition selon l'une des revendications précédentes, caractérisée en ce qu'elle se présente sous forme de gel huileux, d'émulsion eau-dans-huile ou huile-dans-eau ou de dispersion d'huile dans eau grâce à des vésicules.

23. Composition selon l'une des revendications précédentes, caractérisée en ce qu'elle contient, en outre, au moins un ingrédient choisi parmi les parfums, les conservateurs, les cires, les actifs lipophiles.

24. Composition selon l'une des revendications précédentes, caractérisée en ce qu'elle se présente sous forme d'un bâton de rouge à lèvres, de baume à lèvres, de brillant à lèvres ou de composition à appliquer sur un film de rouge à lèvres.

25. Utilisation cosmétique de la composition selon l'une des revendications précédentes pour soigner et/ou maquiller les lèvres.

26. Utilisation de la composition selon l'une des revendications 1 à 24 pour la préparation d'un onguent destiné à traiter les lèvres.

27. Procédé de traitement cosmétique des lèvres, consistant à appliquer sur les lèvres une composition cosmétique telle définie aux revendications 1 à 24.

28. Utilisation, dans une composition contenant une phase grasse liquide et une charge particulaire, de l'association d'au moins une huile comportant une structure chimique pourvue d'au moins un groupement aromatique et d'un épaississant de ladite phase grasse, cet épaississant comprenant au moins un alkyléther de polysaccharide formé de motifs comportant au moins deux cycles osidiques différents, chaque motif comportant au moins un groupe hydroxyle substitué par une chaîne alkyle hydrocarbonée saturée ou non, afin de diminuer la migration de cette phase grasse liquide et/ou obtenir un film brillant.

29. Procédé pour limiter la migration d'une composition de maquillage ou de soin de la peau ou des lèvres sur un substrat différent de la dite peau et desdites lèvres, contenant une phase grasse liquide et une charge particulaire, consistant à introduire dans la phase grasse liquide au moins une huile comportant une structure chimique pourvue d'au moins un groupement aromatique, cette phase grasse étant épaissie par un épaississant comprenant au moins un alkyléther de polysaccharide formé de motifs comportant au moins deux cycles osidiques différents, chaque motif comportant au moins un groupe hydroxyle substitué par une chaîne alkyle hydrocarbonée saturée ou non.

## Patentansprüche

1. Zusammensetzung, die eine flüssige Fettphase, eine partikelförmige Phase und ein Verdickungsmittel für die Fettphase enthält, dadurch gekennzeichnet, daß
das Verdickungsmittel mindestens einen Polysaccharid-alkylether enthält, der aus Einheiten aufgebaut ist, die mindestens zwei unterschiedliche Glykosidringe aufweisen, wobei jede Einheit mindestens eine Hydroxylgruppe trägt, die mit einer gesättigten oder ungesättigten Alkyl-Kohlenwassertoffkette substituiert ist,
und
die Fettphase mindestens ein Öl enthält, dessen chemische Struktur mindestens eine aromatische Gruppe aufweist.

2. Zusammensetzung nach Anspruch 1, dadurch gekennzeichnet, daß zwei bis vier Hydroxylgruppen pro Einheit mit einer gesättigten Alkyl-Kohlenwasserstoffkette substituiert sind.

3. Zusammensetzung nach Anspruch 1 oder 2, dadurch gekennzeichnet, daß die Alkyl-Kohlenwasserstoffkette 1 bis 24 Kohlenstoffatome aufweist.

4. Zusammensetzung nach einem der vorhergehenden Ansprüche, dadurch gekennzeichnet, daß die Alkyl-Kohlenwasserstoffkette 1 bis 10 Kohlenstoffatome aufweist.

5. Zusammensetzung nach einem der vorhergehenden Ansprüche, dadurch gekennzeichnet, daß die Alkylkette ausgewählt ist unter Methyl, Ethyl, n-Propyl, Isopropyl, n-Butyl, Isobutyl und t-Butyl.

6. Zusammensetzung nach einem der vorhergehenden Ansprüche, dadurch gekennzeichnet, daß die Glykosidringe ausgewählt sind unter Mannose, Galactose, Glucose, Furanose, Rhamnose und Arabinose.

7. Zusammensetzung nach einem der vorhergehenden Ansprüche, dadurch gekennzeichnet, daß der Polysaccharid-alkylether ein Alkylether eines Gummis ist, das ausgewählt ist unter Guar-Gummi, Johannisbrotkem-Gummi, Karaya-Gummi und Traganth-Gummi sowie den Gemischen dieser Stoffe.

8. Zusammensetzung nach einem der vorhergehenden Ansprüche, dadurch gekennzeichnet, daß der Alkylether ein Alkylgalactomannan mit einer C₁₋₆-Alkylkette und noch günstiger einer C₁₋₃-Alkylkette ist.

9. Zusammensetzung nach einem der vorhergehenden Ansprüche, dadurch gekennzeichnet, daß der Polysaccharid-alkylether Guar-Gummi mit Ethylketten mit einem Substitutionsgrad von 2 bis 3 ist.

10. Zusammensetzung nach einem der vorhergehenden Ansprüche, dadurch gekennzeichnet, daß der Polysaccharid-alkylether ein Gewichtsmittel des Molekulargewichts von mehr als 200 000 aufweist.

11. Zusammensetzung nach einem der vorhergehenden Ansprüche, dadurch gekennzeichnet, daß der Polysaccharid-alkylether in einer solchen Menge vorliegt, daß das (gewichtsbezogene) Verhältnis der Menge an flüssiger Fettphase zur Menge an Verdikkungsmittel im Bereich von 5 bis 500 gewählt ist.

12. Zusammensetzung nach einem der vorhergehenden Ansprüche, dadurch gekennzeichnet, daß der Polysaccharid-alkylether in einem Mengenanteil von 0,2 bis 16 %, bezogen auf das Gesamtgewicht der Zusammensetzung, und noch günstiger in einem Mengenanteil von 1,5 bis 8 %, bezogen auf das Gesamtgewicht der Zusammensetzung, vorliegt.

13. , Zusammensetzung nach einem der vorhergehenden Ansprüche, dadurch gekennzeichnet, daß das Öl mit einer aromatischen Gruppe ein Kohlenwasserstoffgerüst oder ein Silicongerüst, gegebenenfalls mit fluorierten Gruppen, und mindestens eine Aryl-oder Aralkylgruppe mit 6 bis 60 Kohlenstoffatomen aufweist.

14. Zusammensetzung nach einem der vorhergehenden Ansprüche, dadurch gekennzeichnet, daß das Öl mit einer aromatischen Gruppe ein Kohlenwasserstofföl ist, dessen Struktur mindestens 8 Kohlenstoffatome aufweist.

15. Zusammensetzung nach einem der Ansprüche 1 bis 13, dadurch gekennzeichnet, daß das Öl mit einer aromatischen Gruppe ein Siliconöl der nachstehenden Formel ist: 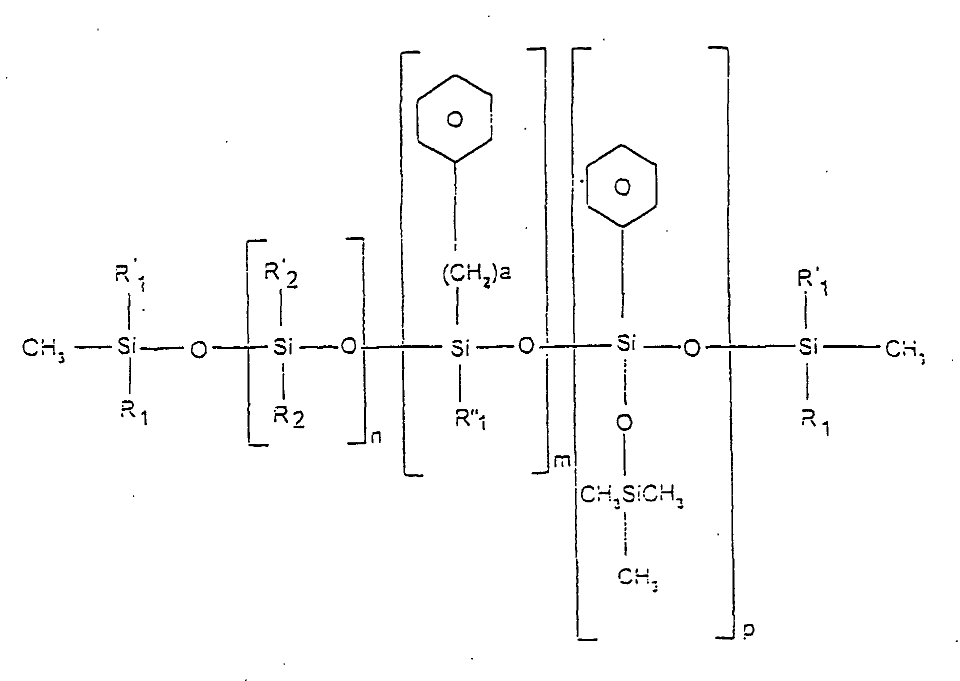 in der bedeuten:
· R₁, R'₁ und R"₁ unabhängig eine geradkettige oder verzweigte, gesättigte oder ungesättigte C₁₋₃₀-Alkylgruppe, eine Arylgruppe, eine Phenylgruppe oder eine C₇₋₆₀-Aralkylgruppe,
· R₂ und R'₂ unabhängig eine geradkettige oder verzweigte, gesättigte oder ungesättigte C₁₋₃₀-Alkylgruppe,
· n eine ganze Zahl von 0 bis 150,
· m eine ganze Zahl von 0 bis 200,
· p eine ganze Zahl von 0 bis 200 und
· a eine ganze Zahl von 0 bis 10
mit der Maßgabe, daß das Verhältnis n/(n + m + p) höchstens gleich 0,85 ist und die Summe n + m + p 1 bis 200 beträgt und R'₁ eine Aryl- oder Aralkylgruppe bedeutet, wenn m = p = 0 ist.

16. Zusammensetzung nach dem vorhergehenden Anspruch, dadurch gekennzeichnet, daß R₁, R'₁ und R"₁ Methyl, Ethyl, Propyl, Isopropyl, Decyl, Dodecyl, Octadecyl, Phenyl, Tolyl, Benzyl oder Phenethyl bedeuten.

17. Zusammensetzung nach Anspruch 15 oder 16, dadurch gekennzeichnet, daß R₂ und R'₂ Methyl, Ethyl, Propyl, Isopropyl, Decyl, Dodecyl oder Octadecyl bedeuten.

18. Zusammensetzung nach einem der vorhergehenden Ansprüche, dadurch gekennzeichnet, daß das Öl mit einer aromatischen Gruppe ausgewählt ist unter den Estern von Trimellitsäure mit C₁₋₂₂-Alkylketten, den Estern von Benzoesäure, den Estern von p-Methoxyzimtsäure, den Estern von Dimethyl-p-aminobenzoesäure, den Estern von Salicylsäure, den Estern von Anthranilsäure, den Phenyltrimethiconen, den Phenyltrimethylsiloxydiphenylsiloxanen, den Diphenylmethyldimethyltrisiloxanen, den Diphenyldimethiconen, den Phenylethyltrimethylsiloxysilicaten, den Phenyltrimethiconen und den Polymethylphenylsiloxanen sowie den Gemischen dieser Stoffe.

19. Zusammensetzung nach einem der vorhergehenden Ansprüche, dadurch gekennzeichnet, daß das Ö1 mit einer aromatischen Gruppe ausgewählt ist unter Tridecyltrimellitat, p-Methoxycinnamat, den Phenyltrimethiconen, den Phenyltrimethylsiloxydiphenylsiloxanen, den Diphenyldimethiconen, den Diphenylmethyldiphenyltrisiloxanen und den 2-Phenylethyltrimethylsiloxysilicaten sowie den Gemischen dieser Stoffe.

20. Zusammensetzung nach einem der vorhergehenden Ansprüche, dadurch gekennzeichnet, daß die Fettphase mindestens ein Öl enthält, das von dem Öl mit einer aromatischen Gruppe verschieden und ausgewählt ist unter Ölen tierischen Ursprungs, Ölen pflanzlichen Ursprungs, Mineralölen und synthetischen Ölen.

21. Zusammensetzung nach einem der vorhergehenden Ansprüche, dadurch gekennzeichnet, daß die partikelförmige Phase in einem Mengenanteil von 0,05 bis 35 %, bezogen auf das Gesamtgewicht der Zusammensetzung, und vorzugsweise in einem Mengenanteil von 0,5 bis 20 % vorliegt.

22. Zusammensetzung nach einem der vorhergehenden Ansprüche, dadurch gekennzeichnet, daß sie in Form eines öligen Gels, einer Wasser-in-Öl-Emulsion oder einer Öl-in-Wasser-Emulsion oder aufgrund von Vesikeln in einer Dispersion von Öl in Wasser vorliegt.

23. Zusammensetzung nach einem der vorhergehenden Ansprüche, dadurch gekennzeichnet, daß sie ferner mindestens einen Bestandteil aufweist, der ausgewählt ist unter Parfums, Konservierungsmitteln, Wachsen und lipophilen Wirkstoffen.

24. Zusammensetzung nach einem der vorhergehenden Ansprüche, dadurch gekennzeichnet, daß sie in Form eines Lippenstifts, eines Lippenbalsams, eines Lippengloss oder einer auf einen Lippenstiftfilm aufzubringenden Zusammensetzung vorliegt.

25. Kosmetische Verwendung der Zusammensetzung nach einem der vorhergehenden Ansprüche zur Pflege und/oder zum Schminken der Lippen.

26. Verwendung der Zusammensetzung nach einem der Ansprüche 1 bis 24 zur Herstellung einer Salbe zur Behandlung der Lippen.

27. Verfahren zur kosmetischen Behandlung der Lippen, das darin besteht, daß eine kosmetische Zusammensetzung nach den Ansprüchen 1 bis 24 auf die Lippen aufgebracht wird.

28. Verwendung der Kombination mindestens eines Öls, dessen chemische Struktur mindestens eine aromatische Gruppe aufweist, und eines Verdickungsmittels für eine Fettphase, das mindestens einen Polysaccharid-alkylether enthält, der aus Einheiten aufgebaut ist, die mindestens zwei unterschiedliche Glykosidringe aufweisen, wobei jede Einheit mindestens eine Hydroxylgruppe aufweist, die mit einer gesättigten oder ungesättigten Alkyl-Kohlenwasserstoffkette substituiert ist, in einer Zusammensetzung, die eine flüssige Fettphase und eine partikelförmige Phase enthält, zur Verringerung der Migration der flüssigen Fettphase und/oder zur Erzielung eines glänzenden Films.

29. Verfahren zur Begrenzung der Migration einer Zusammensetzung zum Schminken oder zur Pflege der Haut oder der Lippen auf einem von der Haut und den Lippen verschiedenen Substrat, die eine flüssige Fettphase und einen partikelförmigen Füllstoff enthält,
das darin besteht, daß in die flüssige Fettphase mindestens ein Öl eingebracht wird, dessen chemische Struktur mindestens eine aromatische Gruppe aufweist, wobei die Fettphase mit einem Verdickungsmittel verdickt ist, das mindestens einen Polysaccharid-alkylether enthält, der aus Einheiten aufgebaut ist, die mindestens zwei unterschiedliche Glykosidringe aufweisen, wobei jede Einheit mindestens eine Hydroxylgruppe trägt, die mit einer gesättigten oder ungesättigten Alkyl-Kohlenwasserstoffgruppe substituiert ist.

## Claims

1. Composition containing a liquid fatty phase, a particulate phase and a thickener for the fatty phase, characterized in that the thickener comprises at least one polysaccharide alkyl ether formed of units containing at least two different saccharide rings, each unit containing at least one hydroxyl group substituted with a saturated or unsaturated hydrocarbon-based alkyl chain, and in that the fatty phase contains at least one oil having a chemical structure containing at least one aromatic group.

2. Composition according to Claim 1, characterized in that two to four hydroxyl groups per unit are substituted with a saturated hydrocarbon-based alkyl chain.

3. Composition according to Claim 1 or 2, characterized in that the hydrocarbon-based alkyl chain contains from 1 to 24 carbon atoms.

4. Composition according to one of the preceding claims, characterized in that the hydrocarbon-based alkyl chain contains from 1 to 10 carbon atoms.

5. Composition according to one of the preceding claims, characterized in that the alkyl chain is chosen from methyl, ethyl, n-propyl, isopropyl, n-butyl, isobutyl and tert-butyl chains.

6. Composition according to one of the preceding claims, characterized in that the saccharide rings are chosen from mannose, galactose, glucose, furanose, rhamnose and arabinose.

7. Composition according to one of the preceding claims, characterized in that the polysaccharide alkyl ether is an alkyl ether of a gum chosen from guar gum, carob gum, karaya gum and gum tragacanth, and mixtures thereof.

8. Composition according to one of the preceding claims, characterized in that the alkyl ether is an alkylated galactomannan with a C₁ to C₆, and better still C₁ to C₃, alkyl chain.

9. Composition according to one of the preceding claims, characterized in that the polysaccharide alkyl ether is a guar gum containing an ethyl chain with a degree of substitution of from 2 to 3.

10. Composition according to one of the preceding claims, characterized in that the polysaccharide alkyl ether has a weight-average molecular weight of greater than 200,000.

11. Composition according to one of the preceding claims, characterized in that the polysaccharide alkyl ether is present in an amount such that the ratio (by weight) of the amount of liquid fatty phase to the amount of thickener is chosen in the range from 5 to 500.

12. Composition according to one of the preceding claims, characterized in that the polysaccharide alkyl ether is present in an amount ranging from 0.2 to 16% of the total weight of the composition, and better still from 1.5 to 8% of the total weight of the composition.

13. Composition according to one of the preceding claims, characterized in that the oil containing an aromatic group contains a hydrocarbon-based or silicone-based skeleton optionally with fluoro groups and at least one aryl or aralkyl radical having from 6 to 60 carbon atoms.

14. Composition according to one of the preceding claims, characterized in that the oil containing an aromatic group is a hydrocarbon-based oil whose structure contains at least 8 carbon atoms.

15. Composition according to one of Claims 1 to 13, characterized in that the oil containing an aromatic group is a silicone oil of formula: 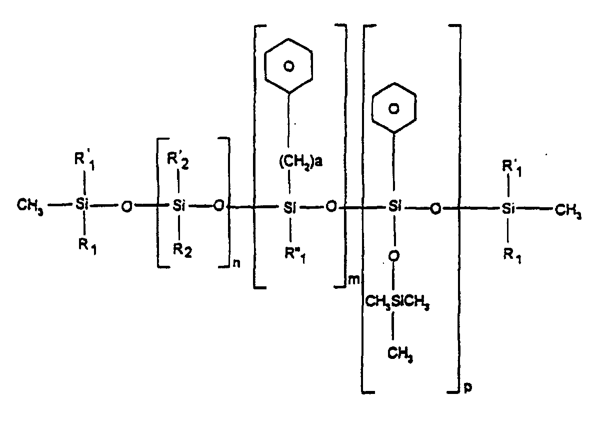 in which
• R₁, R'₁ and R"₁ independently represent a linear or branched, saturated or unsaturated C₁ to C₃₀ alkyl radical, an aryl radical, a phenyl radical or a C₇ to C₆₀ aralkyl radical,
• R₂ and R'₂ independently represent a linear or branched, saturated or unsaturated, C₁ to C₃₀ alkyl radical,
• n is an integer ranging from 0 to 150,
• m is an integer ranging from 0 to 200,
• p is an integer ranging from 0 to 200, and
• a is an integer ranging from 0 to 10,
with the proviso that the ratio n/(n + m + p) is at most equal to 0.85 and that the sum n + m + p ranges from 1 to 200, and that if m = p = 0, then R'₁ is an aryl or aralkyl radical.

16. Composition according to the preceding claim, characterized in that R₁, R'₁ and R"₁ represent a methyl, ethyl, propyl, isopropyl, decyl, dodecyl, octadecyl, phenyl, tolyl, benzyl or phenethyl radical.

17. Composition according to Claim 15 or 16, characterized in that R₂ and R'₂ represent a methyl, ethyl,. propyl, isopropyl, decyl, dodecyl or octadecyl radical.

18. Composition according to one of the preceding claims, characterized in that the oil containing an aromatic group is chosen from trimellitic acid esters containing C₁ to C₂₂ alkyl chains, benzoic acid esters, para-methoxycinnamic acid esters, dimethyl-para-aminobenzoic acid esters, salicylic acid esters, anthranilic acid esters, phenyltrimethicones, phenyltrimethylsiloxydiphenylsiloxanes, diphenylmethyldimethyltrisiloxanes, diphenyldimethicones, phenyl ethyl trimethylsiloxysilicates, phenyltrimethicones and polymethylphenylsiloxanes, and mixtures thereof.

19. Composition according to one of the preceding claims, characterized in that the oil containing an aromatic group is chosen from tridecyl trimellitate, para-methoxycinnamate, phenyltrimethicones, phenyltrimethylsiloxydiphenylsiloxanes, diphenyldimethicones, diphenylmethyldiphenyltrisiloxanes and 2-phenylethyl trimethylsiloxysilicates, and mixtures thereof.

20. Composition according to one of the preceding claims, characterized in that the fatty phase contains at least one oil other than the oil containing an aromatic group, chosen from oils of animal, plant, mineral and synthetic origin.

21. Composition according to one of the preceding claims, characterized in that the particulate phase is present in a proportion of from 0.05 to 35% of the total weight of the composition, preferably from total weight of the composition, preferably from 0.5 to 20%.

22. Composition according to one of the preceding claims, characterized in that it is in the form of an oily gel, a water-in-oil or oil-in-water emulsion or a dispersion of oil in water by means of vesicles.

23. Composition according to one of the preceding claims, characterized in that it also contains at least one ingredient chosen from fragrances, preserving agents, waxes and lipophilic active agents.

24. Composition according to one of the preceding claims, characterized in that it is in the form of a tube of lipstick, a lip balm, a lip gloss or a composition to be applied to a film of lipstick.

25. Cosmetic use of the composition according to one of the preceding claims to care for and/or make up the lips.

26. Use of the composition according to one of Claims 1 to 24 for the preparation of an ointment intended to treat the lips.

27. Cosmetic treatment process for the lips, which consists in applying a cosmetic composition as defined in Claims 1 to 24 to the lips.

28. Use, in a composition containing a liquid fatty phase and a particulate filler, of a combination of at least one oil having a chemical structure containing at least one aromatic group, and of a thickener for the said fatty phase, this thickener comprising at least one polysaccharide alkyl ether formed of units containing at least two different saccharide rings, each unit containing at least one hydroxyl group substituted with a saturated or unsaturated hydrocarbon-based alkyl chain, in order to reduce the migration of this liquid fatty phase and/or to obtain a glossy film.

29. Process for limiting the migration of a make-up or care composition for the skin or the lips on a substrate other than the said skin and the said lips, containing a liquid fatty phase and a particulate filler, this process consisting in introducing into the liquid fatty phase at least one oil having a chemical structure containing at least one aromatic group, this fatty phase being thickened by a thickener comprising at least one polysaccharide alkyl ether formed of units containing at least two different saccharide rings, each unit containing at least one hydroxyl group substituted with a saturated or unsaturated hydrocarbon-based alkyl chain.
